# EUROPÄISCHE PATENTSCHRIFT

(11) **EP 2 946 718 B1**
(45) Veröffentlichungstag und Bekanntmachung des Hinweises auf die Patenterteilung: **31.01.2018**
(21) Anmeldenummer: 15167503.0
(22) Anmeldetag: 13.05.2015
(51) Int. Cl.: A61B 1/00, A61B 1/06, A61B 1/12, G02B 23/24

(54) **OPTISCHES MEDIZINISCHES INSTRUMENT, INSBESONDERE ENDOSKOP ODER EXOSKOP**
OPTICAL MEDICAL INSTRUMENT, PARTICULARLY AN ENDOSCOPE OR EXOSCOPE
INSTRUMENT OPTIQUE MÉDICAL, EN PARTICULIER ENDOSCOPE OU EXOSCOPE

(30) Priorität: 22.05.2014 DE 102014107205
(43) Veröffentlichungstag der Anmeldung: 25.11.2015
(73) Patentinhaber: Karl Storz SE & Co. KG, 78532 Tuttlingen (DE)
(72) Erfinder: Pilz, Kevin, 78532 Tuttlingen (DE); Kupferschmid, Markus, 78576 Emmingen-Liptingen (DE); Ulmschneider, Daniel, 78532 Nendingen (DE); Heni, Andreas, 78567 Fridingen (DE)
(74) Vertreter: Witte, Weller & Partner Patentanwälte mbB

(56) Entgegenhaltungen:
- JP-A- 2009 011 612
- US-A- 5 207 674
- US-A1- 2006 195 165
- US-A1- 2008 151 046

## Beschreibung

Die Erfindung betrifft ein optisches medizinisches Instrument, insbesondere ein Endoskop oder Exoskop, mit einem langerstreckten rohrförmigen thermisch leitenden Schaft, mit einer Verlustwärme erzeugenden Wärmequelle, mit einer Heatpipe, die sich im Inneren des Schaftes in Längsrichtung des Schaftes erstreckt und ein distales Heatpipeende und ein proximales Heatpipeende aufweist, wobei die Heatpipe mit der Wärmequelle thermisch gekoppelt ist, um von dieser Wärme aufzunehmen und von der Wärmequelle abzuführen.

Ein solches optisches medizinisches Instrument ist aus DE 10 2010 024 003 A1 und US 2008/151046 bekannt.

Endoskope werden im Rahmen der minimal-invasiven Chirurgie als Beobachtungsinstrumente verwendet. Bei einem Endoskop wird der langerstreckte Schaft durch eine künstlich geschaffene oder natürliche Öffnung teilweise in den Körper eingeführt.

Ein Exoskop ist ein Beobachtungsinstrument, das zur Bildgebung bei offenen chirurgischen Eingriffen eingesetzt wird. Ein Exoskop wird vollständig extrakorporal in einem Abstand zum Operationsfeld mittels eines Haltearms platziert.

Sowohl Endoskope als auch Exoskope sind heutzutage häufig als Video-Endoskope bzw. Video-Exoskope ausgeführt und entsprechend mit elektronischen Bauelementen wie beispielsweise Bildsensoren, einer Steuerelektronik für die Bildsensoren und/oder Lichtquellen wie LEDs zur Beleuchtung bestückt. Die elektronischen Bauelemente erzeugen im Betrieb Verlustwärme und stellen somit eine oder mehrere Wärmequellen dar. Die Verlustwärme muss von der oder den Wärmequellen über einen Wärmeübergang an die Umgebung abgeführt werden. Die Abfuhr der Wärme ist zum Schutz der elektronischen Bauelemente gegen übermäßige Erwärmung und dadurch verursachten Leistungsabfall und sogar Beschädigung erforderlich. Andererseits darf bei einem Endoskop zumindest der in den Körper eingeführte Abschnitt des Schaftes nicht so stark erwärmt werden, dass Verletzungen des Patienten zu befürchten sind. Ein Endoskop, das in der Chirurgie verwendet wird, unterliegt Vorschriften des Medizinproduktgesetzes. Gemäß dem Medizinproduktgesetz darf der Endoskopschaft außenseitig eine Temperatur von 41 °C nicht überschreiten, um eine wärmebedingte Beschädigung von Gewebe im menschlichen oder tierischen Körper zu vermeiden.

Ohne ein eigenes Wärmeabfuhrmanagement erfolgt der Wärmeübergang von der Wärmequelle auf die Umgebung über freie Konvektion oder thermische Strahlung. Für diesen Wärmeübergang steht als Schnittstelle zur Umgebung die Oberfläche des Instrumentes zur Verfügung. Bedingt durch die lokale Position der Wärmequelle oder Wärmequellen wird die Oberfläche jedoch nicht gleichmäßig erhitzt. Es treten lokale Temperaturmaxima und -minima auf. Die Oberfläche des Instruments ist somit nicht gleichmäßig gut in den Prozess der Wärmeabfuhr an die Umgebung eingebunden. Besonders die Stellen der Temperaturmaxima überschreiten des Weiteren die zulässigen Temperaturen, während das Potenzial der Temperaturminima ungenutzt bleibt. Durch die Zunahme leistungsstarker Elektronik in optischen medizinischen Instrumenten wie Endoskopen und Exoskopen wird dieser punktuelle Wärmeübergang zukünftig auch nicht mehr genügen, um die Elektronik hinreichend zu kühlen.

Ein weiterer Nachteil ist ein aus dem ungleichmäßigen Wärmeübergang zur Umgebung entstehender hoher Temperaturgradient, also eine hohe Temperaturdifferenz zwischen Wärmequelle und Umgebung. Die hohen Temperaturen an der Wärmequelle, d.h. der Elektronik, beeinflussen die Leistung der Elektronik negativ.

In dem eingangs genannten Dokument ist ein Endoskop beschrieben, das eine Heatpipe aufweist, um die von einer im distalen Bereich des Schafts angeordneten Lichtquelle erzeugte Verlustwärme nach proximal abzuführen, wobei sich die Heatpipe bis in ein Kopfstück am proximalen Ende des Schaftes erstreckt, wobei in dem Kopfstück ein Wärmesenkekörper angeordnet ist, mit dem die Heatpipe thermisch gekoppelt ist, der die Verlustwärme von der Heatpipe aufnimmt und direkt oder über das Gehäuse des Kopfstücks an die Umgebung abgibt. Um zu vermeiden, dass sich das distale Ende des Schafts über die maximal zulässige Temperatur von 41 °C aufheizt, ist des Weiteren vorgesehen, dass der Schaft innenseitig gegen die Heatpipe thermisch isoliert ist.

Dieses Wärmeabfuhrmanagement des bekannten Endoskops stößt bei einer leistungsstarken und somit viel Verlustwärme erzeugenden Elektronik als Wärmequelle an seine Grenzen, und gewährleistet insbesondere, wenn sich die Wärmequelle im distalen Bereich des Schaftes befindet, keine hinreichende Wärmeabfuhr von der Wärmequelle.

In US 2008/0151046 A1 ist des Weiteren ein Endoskop beschrieben, bei dem zur Abfuhr von Verlustwärme einer im distalen Bereich des Schafts angeordneten Lichtquelle eine Heatpipe vorhanden ist, die sich von der distalseitigen Lichtquelle, mit der die Heatpipe thermisch gekoppelt ist, über eine kleine Teillänge des Schafts nach proximal erstreckt, wobei mit dem proximalen Ende der Heatpipe ein wärmeleitfähiger Draht, beispielsweise ein Kupferdraht, verbunden ist, wobei der Draht sich weiter nach proximal erstreckt und mit seinem proximalen Ende mit der Innenseite des Schafts des Endoskops wärmeleitend verbunden ist. Über das Ende des Drahtes wird die Wärme lediglich punktuell auf den Endoskopschaft übertragen, wodurch die Wärmeabfuhr ebenfalls nur punktuell an die Umgebung erfolgt, was jedoch für größere Wärmemengen eine unzureichende Wärmeabfuhr darstellt.

US 2006/0195165 A1 offenbart ein optisches Therapieinstrument, mit dem therapeutisches Licht einem Körperhohlraum zugeführt wird. Das Instrument weist als Lichtquellen LEDs auf, die Wärme erzeugen. Damit die Wärme nicht dem Körperhohlraum zugeführt wird, in den das Instrument eingeführt ist, sind bei dem Instrument Maßnahmen zur Wärmeabfuhr vorgesehen, wie beispielsweise Heatpipes.

JP 2009 011612 A offenbart ein Endoskop mit einem Schaft, an dessen distalem Ende eine LED-Lichtquelle angeordnet ist sowie eine Wärmesenke, die mit der LED-Lichtquelle thermisch verbunden ist. Ein Wärmeübertragungssystem ist dazu vorgesehen, Wärme, die von der Wärmesenke aufgenommen wird, nach proximal zu führen.

US 5 207 674 A offenbart ein Instrument für die Kryochirurgie zum Vereisen von Hautzellen. Das Instrument weist eine Heatpipe zur Wärmeabfuhr auf.

Der Erfindung liegt die Aufgabe zugrunde, ein optisches medizinisches Instrument der eingangs genannten Art dahingehend weiterzubilden, dass die Abfuhr von Verlustwärme zur Umgebung des Instruments verbessert ist.

Hinsichtlich des eingangs genannten optischen medizinischen Instruments wird diese Aufgabe dadurch gelöst, dass die Heatpipe zwischen dem distalen Heatpipeende und dem proximalen Heatpipeende über zumindest eine Teillänge der Heatpipe und über zumindest einen Teilumfang der Heatpipe mit dem Schaft flächig thermisch leitend gekoppelt ist, um Wärme von der Heatpipe über zumindest eine Teillänge und zumindest einen Teilumfang des Schaftes an die Umgebung abzuführen, wobei sich die Heatpipe zumindest über die halbe Länge des Schaftes erstreckt und über zumindest die halbe Länge des Schaftes mit diesem thermisch leitend gekoppelt ist.

Im Unterschied zum Stand der Technik nutzt die vorliegende Erfindung den Schaft des Instruments für einen Wärmeübergang zur Umgebung, jedoch nicht punktuell, sondern großflächig. Bei dem erfindungsgemäßen Instrument wird somit der Schaft des Instruments selbst als Wärmesenke genutzt. Im Stand der Technik wurde des Weiteren davon ausgegangen, dass eine Heatpipe nur an dem einen Ende Wärme aufnehmen und an dem entgegengesetzten Ende Wärme abgeben kann. Es hat sich jedoch im Rahmen der Erfindung herausgestellt, dass eine Heatpipe auch zwischen ihren beiden Enden Wärme abgeben kann. Wenn, wie dies erfindungsgemäß vorgesehen ist, der Schaft des Instruments als Ganzes oder zumindest ein größerer Bereich des Schaftes als Wärmesenke genutzt wird, wird die Heatpipe die von der Wärmequelle abgeführte Wärme dort abgeben, wo sich gerade die kälteste Stelle des Schaftes befndet. Je größer der Bereich und die Fläche der thermisch leitenden Kopplung zwischen der Heatpipe und dem Schaft ist, kann sich die Wärmesenke entlang der Heatpipe und des Schaftes an desto mehr Stellen, mitunter überall entlang der Heatpipe befinden. Entsprechend der Funktionsweise von Heatpipes stellt sich also die Position der Wärmesenke "flexibel" entlang des Schaftes von selbst ein. Dieses erfindungsgemäße Konzept wird nachfolgend als "flexible Wärmesenke" bezeichnet. Durch das Konzept der "flexiblen Wärmesenke" wird erreicht, dass sich die Wärme über die vorhandene Oberfläche des Schaftes relativ gleichmäßig verteilt und Temperaturdifferenzen somit so gering wie möglich gehalten werden. Vorteilhafterweise werden dadurch lokale Temperaturspitzen und somit hohe Temperaturgradienten entlang des Schaftes zumindest verringert oder gar vermieden. Die Verlustwärme wird vielmehr gleichmäßig von der Heatpipe auf den Schaft und von diesem an die Umgebung übertragen. Die Gefahr eines Aufheizens des Schafts über die vom Medizinproduktegesetz vorgesehene Grenze von maximal 41 °C wird somit ebenfalls vermieden. Die Wärmeabfuhr bei dem erfindungsgemäßen Instrument ist auch sehr effizient, da der Wärmeübergang über eine große Oberfläche des Schaftes erfolgt.
Weiterhin im Sinne der Ausnutzung des Prinzips der "flexiblen Wärmesenke," und im Sinne eines noch weiter verbesserten gleichmäßigeren Wärmeübergangs von der Heatpipe zum Schaft erstreckt sich die Heatpipe zumindest über die halbe Länge des Schaftes und ist über zumindest die halbe Länge des Schaftes mit diesem thermisch leitend gekoppelt.

Es versteht sich, dass der Schaft des erfindungsgemäßen Instruments innenseitig nicht gegenüber der Heatpipe thermisch isoliert ist, sondern gerade im Gegenteil thermisch möglichst gut leitend mit der Heatpipe gekoppelt ist. Der Schaft selbst ist ebenfalls thermisch leitend, d.h. weist ein thermisch leitendes Material auf, wie bspw. Edelstahl. Ebenso weist die Heatpipe außenseitig keine Isolierung auf und kann insbesondere ein Rohr aus einem gut wärmeleitenden Material, beispielsweise Kupfer oder Aluminium, aufweisen.

In einer bevorzugten Ausgestaltung ist die Heatpipe im Sinne der vorstehend genannten "flexiblen Wärmesenke" über zumindest die halbe Gesamtlänge der Heatpipe mit dem Schaft thermisch leitend gekoppelt, und weiter vorzugsweise ist die Heatpipe nahezu oder vollständig über die Gesamtlänge der Heatpipe mit dem Schaft thermisch leitend gekoppelt.

Sofern es die Platzverhältnisse im Schaft des Instruments zulassen, ist es optimal, wenn die Heatpipe oder eine Anordnung mehrerer Heatpipes sich über deutlich mehr als die halbe Länge des Schaftes, vorzugsweise über mehr als drei Viertel der Länge des Schaftes erstreckt und entlang dieses Bereichs mit dem Schaft thermisch leitend gekoppelt ist.

Anstelle einer einzelnen langen durchgehenden Heatpipe kann die Heatpipe auch zumindest zwei Heatpipes aufweisen, die in Längsrichtung des Schaftes hintereinander, ggf. mit einem teilweisen Überlapp und/oder mit thermisch leitender Kopplung untereinander, angeordnet sind.

Die Heatpipe kann zumindest zum Teil unmittelbar mit dem Schaft thermisch gekoppelt sein, wobei die Heatpipe zumindest teilumfänglich und vorzugsweise formschlüssig mit einer Innenseite des Schaftes in Berührung steht, wie in einer bevorzugten Ausgestaltung vorgesehen ist, und/oder die Heatpipe kann zumindest teilumfänglich über einen Wärmesenkekörper mit dem Schaft thermisch leitend gekoppelt sein, wobei der Wärmesenkekörper mit der Heatpipe und mit einer Innenseite des Schaftes jeweils zumindest teilumfänglich formschlüssig in Berührung steht, wie in einer weiteren bevorzugten Ausgestaltung vorgesehen ist.

Wesentlich für die Wirksamkeit des erfindungsgemäßen Wärmeabfuhrmanagements ist die thermisch leitende Anbindung der Heatpipe an den Schaft über eine möglichst große Fläche, sei es bei der unmittelbaren Anbindung der Heatpipe an die Innenseite des Schaftes oder sei es alternativ oder zusätzlich über einen oder mehrere Wärmesenkekörper. Der Formschluss zwischen der Heatpipe und der Innenseite des Schaftes bei unmittelbarer thermisch leitender Kopplung und der Formschluss zwischen Wärmesenkekörper und Heatpipe einerseits und Wärmesenkekörper und Innenseite des Schaftes andererseits im Falle einer mittelbaren thermisch leitenden Kopplung der Heatpipe mit dem Schaft verbessert die thermische Anbindung der Heatpipe an den Schaft.

Im Zusammenhang der mittelbaren thermisch leitenden Anbindung der Heatpipe an den Schaft ist es auch bevorzugt, wenn die Heatpipe zumindest teilumfänglich über eine Mehrzahl an Wärmesenkekörpern mit dem Schaft thermisch leitend gekoppelt ist, wobei die Wärmesenkekörper entlang der Heatpipe verteilt angeordnet sind.

In einer weiteren bevorzugten Ausgestaltung ist die Heatpipe mit der Wärmequelle über ein thermisch leitendes Wärmekoppelelement verbunden, mit dem die Heatpipe vorzugsweise formschlüssig verbunden ist.

Gegenüber einer auf Wärmestrahlung oder Konvektion beruhenden thermischen Kopplung zwischen Heatpipe und Wärmequelle hat die thermisch leitende Kopplung den Vorteil eines wirksameren Wärmeübergangs von der Wärmequelle auf die Heatpipe, insbesondere wenn die Verbindung der Heatpipe mit dem Wärmekoppelelement formschlüssig ist.

Im Zusammenhang mit der vorstehend genannten Ausgestaltung ist es bevorzugt, wenn die Heatpipe in das Wärmekoppelelement eingepresst ist.

Durch das Einpressen der Heatpipe in das Wärmekoppelelement wird auf einfach herzustellende Weise eine formschlüssige Verbindung zwischen der Heatpipe und dem Wärmekoppelelement realisiert, wobei durch das Einpressen die Heatpipe auch im Querschnitt verformt werden kann.

Wenn ein übermäßiges Verformen der Heatpipe, das die innere Kapillarwirkung der Heatpipe beeinträchtigen kann, vermieden werden soll, kann die formschlüssige Verbindung der Heatpipe mit dem Wärmekoppelelement auch mittels eines thermisch leitenden Fügematerials zusätzlich fixiert sein.

In diesem Fall erfolgt die Anbindung der Heatpipe an das Wärmekoppelelement formschlüssig mit einer Spielpassung, wobei der durch das Passungsspiel bedingte Freiraum und verbleibende Freiheitsgrade durch das zusätzliche Fügen, beispielsweise Kleben oder Löten, kompensiert werden. Wenn allerdings dem thermischen Ausdehnungsverhalten des Wärmekoppelelements oder der Heatpipe durch entsprechende frei zu bleibende Freiheitsgrade Rechnung getragen werden muss, entfällt die zusätzliche stoffschlüssige Verbindung.

Ebenso ist es gemäß einer weiteren bevorzugten Ausgestaltung möglich, dass das Wärmekoppelelement als thermisch leitendes Lager mit zumindest einem Bewegungsfreiheitsgrad zur Wärmequelle ausgebildet ist.

Diese Maßnahme ist insbesondere dann von Vorteil, wenn die Wärmequelle eine bewegliche Wärmequelle ist, beispielsweise ein verschwenkbar angeordneter Bildsensor im Falle einer verschwenkbaren Kamera. In diesem Fall werden in dem Wärmekoppelelement die Funktionen thermisches Leiten und bewegliches Lagern ineinander integriert. Die Heatpipe selbst ist unbeweglich im Schaft angeordnet.

Gemäß einer weiteren Ausgestaltung der Erfindung ist die Wärmequelle in einem distalen Endbereich des Schaftes, in einem mittleren Abschnitt oder in einem proximalen Endbereich des Schaftes angeordnet.

Das erfindungsgemäße Prinzip des Wärmeabfuhrmanagements ist unabhängig davon wirksam, wo sich die Wärmequelle oder die Wärmequellen befindet bzw. befinden. Für die Wärmeabfuhr gibt es aufgrund des erfindungsgemäßen Prinzips der "flexiblen Wärmesenke" keine Vorzugsrichtung der Wärmeabfuhr. Vielmehr kann die Wärmeabfuhr von distal nach proximal, von proximal nach distal oder von einem mittleren Bereich des Schaftes nach distal und/oder proximal erfolgen, je nachdem, wo sich gerade die kälteste Stelle der Heatpipe bzw. des Schaftes befindet.

In entsprechender Weise ist die Heatpipe gemäß einer weiteren Ausgestaltung mit dem distalen Heatpipeende, mit einem mittleren Abschnitt zwischen dem distalen Heatpipeende und dem proximalen Heatpipeende oder mit dem proximalen Heatpipeende mit der Wärmequelle thermisch gekoppelt.

Aufgrund des erfindungsgemäßen Prinzips der Wärmeabfuhr sucht sich das Arbeitsmedium in der Heatpipe den gerade bzw. temporär kältesten Punkt der Heatpipe als Wärmesenke und den temporär wärmsten Punkt an der Heatpipe als Wärmequelle ("flexible Wärmesenke"). Es ist daher im Rahmen der Erfindung gerade nicht erforderlich, dass die Heatpipe mit einem Ende mit der Wärmequelle und mit dem anderen Ende mit der Wärmesenke verbunden ist.

Wie bereits erwähnt, ist es im Rahmen der Erfindung möglich, dass die Heatpipe nicht nur mit einer Wärmequelle, sondern auch mit mehreren Wärmequellen thermisch gekoppelt sein kann, wobei diese Wärmequellen in Längsrichtung des Schaftes voneinander beabstandet sind.

Ebenso ist es möglich, nicht nur eine Heatpipe mit der oder den Wärmequellen thermisch leitend zu koppeln, sondern es können im Inneren des Schaftes auch mehrere Heatpipes angeordnet sein, die mit der einen oder den mehreren Wärmequellen thermisch leitend gekoppelt sind.

Die mehreren Heatpipes können im Schaft nebeneinander verlaufen oder in Längsrichtung hintereinander, wie bereits oben erwähnt wurde.

Die Heatpipe ist gemäß einer weiteren bevorzugten Ausgestaltung eine gesinterte Heatpipe.

Bei einer gesinterten Heatpipe, die beispielsweise aus Kupfer besteht, sind an der Innenseite des Rohrs der Heatpipe gesinterte Kupferpartikel vorhanden. Gesinterte Heatpipes sind gravitationsunabhängiger als andere Bauarten von Heatpipes, d.h. ihre Wirksamkeit hängt nicht entscheidend davon ab, ob sie sich parallel oder quer zur Schwerkraft erstrecken.

Im Rahmen der Erfindung ist es des Weiteren vorteilhaft, wenn mehrere dünne Heatpipes verwendet werden anstatt einer Heatpipe mit großem Durchmesser. Dünne Heatpipes haben neben einer ebenfalls geringeren Lageabhängigkeit den Vorteil eines größeren Oberflächen-Volumen-Verhältnisses als eine Heatpipe mit einem größeren Durchmesser.

Weitere Vorteile und Merkmale ergeben sich aus der nachfolgenden Beschreibung und der beigefügten Zeichnung.

Es versteht sich, dass die vorstehend genannten und nachstehend noch zu erläuternden Merkmale nicht nur in der jeweils angegebenen Kombination, sondern auch in anderen Kombinationen oder in Alleinstellung verwendbar sind, ohne den Rahmen der vorliegenden Erfindung zu verlassen.

Ausführungsbeispiele der Erfindung sind in der Zeichnung dargestellt und werden mit Bezug auf diese hiernach näher beschrieben. Es zeigen:
- Fig. 1: eine Prinzipskizze eines optischen medizinischen Instruments im Längsschnitt gemäß einem Ausführungsbeispiel;
- Fig. 2: eine Prinzipskizze eines optischen medizinischen Instruments im Längsschnitt gemäß einem weiteren Ausführungsbeispiel;
- Fig. 3: eine Prinzipskizze eines optischen medizinischen Instruments im Längsschnitt gemäß einem weiteren Ausführungsbeispiel;
- Fig. 4: eine Prinzipskizze eines optischen medizinischen Instruments im Längsschnitt gemäß einem weiteren Ausführungsbeispiel;
- Fig. 5: eine Prinzipskizze eines optischen medizinischen Instruments im Längsschnitt gemäß einem weiteren Ausführungsbeispiel;
- Fig. 6: eine Prinzipskizze eines optischen medizinischen Instruments im Längsschnitt gemäß einem weiteren Ausführungsbeispiel;
- Fig. 7: eine Prinzipskizze eines optischen medizinischen Instruments im Längsschnitt gemäß einem weiteren Ausführungsbeispiel;
- Fig. 8: eine Prinzipskizze eines optischen medizinischen Instruments im Längsschnitt gemäß einem weiteren Ausführungsbeispiel;
- Fig. 9: eine Prinzipskizze eines medizinischen optischen Instruments im Längsschnitt gemäß einem weiteren Ausführungsbeispiel;
- Fig. 10: eine Prinzipskizze eines optischen medizinischen Instruments im Längsschnitt gemäß einem weiteren Ausführungsbeispiel;
- Fig. 11: eine Prinzipskizze eines optischen medizinischen Instruments gemäß einem weiteren Ausführungsbeispiel;
- Fig. 12: eine Prinzipskizze eines optischen medizinischen Instruments im Längsschnitt gemäß einem weiteren Ausführungsbeispiel;
- Fig. 13: eine Prinzipskizze eines optischen medizinischen Instruments im Längsschnitt gemäß einem weiteren Ausführungsbeispiel;
- Fig. 14: eine schematische Seitenansicht eines optischen medizinischen Instruments gemäß einem weiteren Ausführungsbeispiel;
- Fig. 15: einen Abschnitt des Instruments in Fig. 14 mit teilweise aufgebrochenem Schaft;
- Fig. 16: einen Schnitt durch das Instrument in Fig. 14 entlang einer Linie XVI-XVI in Fig. 14 und in gegenüber Fig. 14 vergrößertem Maßstab; und
- Fig. 17: einen Schnitt ähnlich zu Fig. 16 durch einen Schaft eines optischen medizinischen Instruments gemäß einem weiteren Ausführungsbeispiel.

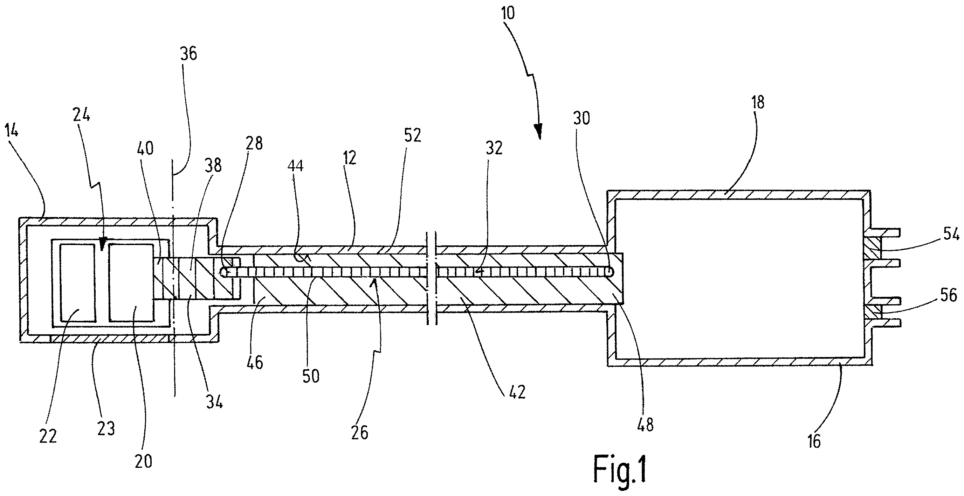

Fig. 1 zeigt ein optisches medizinisches Instrument 10 in einer Prinzipdarstellung. Das Instrument 10 ist ein Endoskop oder ein Exoskop.

Allgemein weist das Instrument 10 einen langerstreckten rohrförmigen Schaft 12 zwischen einem distale Ende 14 und einem proximalen Ende 16 aufweist. Der Schaft 12 ist unterbrochen gezeigt, um anzudeuten, dass sich der Schaft 12 über eine große Länge erstrecken kann. In Fig. 1 ist der distale Endbereich des Instruments 10 und der proximale Endbereich des Instruments 10 des Weiteren verbreitert gezeigt, was jedoch lediglich beispielhaft zu verstehen ist. Das proximale Ende 16 ist hier als Instrumentenkopfgehäuse 18 ausgebildet. Der Schaft 12 und das Instrumentenkopfgehäuse 18 stellen das Gehäuse für die darin befindlichen Komponenten dar. Das Instrumentenkopfgehäuse 18 kann zum Schaft 12 gehören. Ebenso ist der Schaft 12 selbst als Gehäuse zu verstehen. Das Gehäuse kann hermetisch dicht sein.

Zu den Komponenten im Schaft 12 gehören im distalen Endbereich des Schafts 12 angeordnete elektronische Bauelemente wie ein oder mehrere Bildsensoren, bestückte Platinen und dgl., die in Fig. 1 unter dem Bezugszeichen 20 zusammengefasst sind und nachfolgend als Elektronik 20 bezeichnet werden.

Weitere Komponenten im distalen Endbereich dienen zur Beleuchtung und sind unter dem Bezugszeichen 22 zusammengefasst und werden nachfolgend als Beleuchtung 22 bezeichnet. Die Beleuchtung 22 kann durch eine oder mehrere Lichtquellen wie LEDs oder durch lichtaustrittsseitige Enden von Glasfasern gebildet sein.

Ein Fenster 23, das im gezeigten Ausführungsbeispiel parallel zur Längsrichtung des Schaftes 12 verläuft, dient dem Lichtaustritt für die Beleuchtung 22 und dem Lichteintritt zu dem oder den Bildsensoren der Elektronik 20. Während im gezeigten Ausführungsbeispiel die Blickrichtung des Instruments 10 somit 90° zur Längsrichtung des Schaftes 12 ist, versteht es sich, dass auch andere Blickrichtungen wie 0°, 30°, 60° und dgl., wie sie insbesondere bei Endoskopen üblich sind, möglich sind.

Die Elektronik 20 und gegebenenfalls die Beleuchtung 22 stellen eine Verlustwärme erzeugende Wärmequelle 24 dar. Insbesondere die Elektronik 20 erzeugt eine große Verlustwärmemenge, die von der Elektronik 20 abgeführt werden muss, da bekanntlich die Leistungsfähigkeit von elektronischen Bauelementen mit zunehmender Temperatur sinkt.

Um die Verlustwärme von der Wärmequelle 24 effizient abzuführen, weist das Instrument 10 ein Wärmeabfuhrmanagement auf, das nachfolgend beschrieben wird.

Hierzu gehört eine Heatpipe 26, die sich im Inneren des Schaftes 12 in einem langerstreckten Bereich zwischen dem distalen Ende 14 und dem proximalen Ende 16 erstreckt und ein distales Heatpipeende 28 und ein proximales Heatpipeende 30 aufweist.

Die Heatpipe 26 ist ein Wärmerohr, das ein hermetisch gekapseltes Volumen einschließt, das mit einem Arbeitsmedium, beispielsweise Wasser oder Ammoniak, gefüllt ist, das das Volumen zu einem kleinen Teil in flüssigem, zum größeren in dampfförmigem Zustand ausfüllt. Bei Wärmeeintrag in die Heatpipe 26 beginnt das Arbeitsmedium zu verdampfen, wobei der entstandene Dampf von der Stelle des Wärmeeintrags wegströmt. Die Heatpipe 26 ist an ihrer äußeren Oberfläche über die gesamte Länge der Heatpipe 26 nicht thermisch isoliert.

Als Heatpipe 26 können alle gängigen Typen von Heatpipes verwendet werden, wobei im vorliegenden Fall die Ausgestaltung der Heatpipe 26 als gesinterte Heatpipe bevorzugt ist. Die Funktion gesinterter Heatpipes ist im Unterschied zu anderen Typen von Heatpipes weniger lageabhängig in Bezug auf die Gravitation. Des Weiteren sollte die Heatpipe 26 im Querschnitt nicht zu groß sein, sondern eher schmal, da dies ebenfalls die Lageabhängigkeit der Heatpipe 26 in Bezug auf Gravitation weiter verringert und außerdem das Oberflächen-Volumen-Verhältnis größer und damit günstiger ist als bei einer dickeren Heatpipe. Ein größeres Oberflächen-Volumen-Verhältnis trägt zur Verbesserung des Wärmeübergangs von der Heatpipe 26 in ihre Umgebung bei. Die Heatpipe 26 kann einen runden Querschnitt, aber auch einen ovalen oder abgeflachten Querschnitt aufweisen.

Die Heatpipe 26 ist mit der Wärmequelle 24 thermisch, hier leitend gekoppelt, um von der Wärmequelle 24 Wärme aufzunehmen und von der Wärmequelle 24 abzuführen.

Dazu ist die Heatpipe 26, im vorliegenden Ausführungsbeispiel das distale Heatpipeende 28, über ein Wärmekoppelelement 34 thermisch leitend mit der Wärmequelle 24 verbunden.

Das Wärmekoppelelement 34 kann in Form eines Wärmeleitbleches oder Wärmeleitkörpers aus einem thermisch gut leitenden Material, beispielsweise Kupfer oder Aluminium, gebildet sein. Die Anbindung der Wärmequelle 24 an das Wärmekoppelelement 34 kann durch Einbringung einer Graphitfolie (nicht gezeigt) verbessert werden.

Im Fall, dass das Instrument 10 beispielsweise ein Video-Exoskop oder ein Video-Endoskop mit verschwenkbarer Kamera (Bildsensor oder Bildsensoren der Elektronik 20) ist, wobei die Kamera beispielhaft um eine Schwenkachse 36 verschwenkbar ist, ist das Wärmekoppelelement 34 als thermisch leitendes Lager mit einem Bewegungsfreiheitsgrad einer Drehung um die Schwenkachse 36 ausgebildet. In diesem Fall kann das Wärmekoppelelement 34 aus einem feststehenden Teil 38 und einem relativ dazu beweglichen (drehbaren) Teil 40 ausgebildet sein, wobei das bewegliche Teil 40 mit der Wärmequelle 24 verbunden ist. Es versteht sich, dass die Verbindung zwischen dem feststehenden Teil 38 und dem relativ dazu beweglichen Teil 40 des Wärmekoppelelements 34 thermisch gut leitend sein muss. Dies kann durch ein wärmeleitendes Zwischenmaterial zwischen den Lagerhälften (Teile 38 und 40), beispielsweise Graphitfolie, bewerkstelligt bzw. verbessert werden. Es versteht sich, dass die Schwenkachse der Kamera auch mit der Blickachse des Instruments 10 übereinstimmen kann.

Die Heatpipe 26 ist mit dem Wärmekoppelelement 34 insbesondere formschlüssig verbunden, wobei dies dadurch realisiert sein kann, dass im vorliegenden Fall das distale Heatpipeende 28 in eine Ausnehmung. des Wärmekoppelelements 34 eingepresst ist. Die Anbindung der Heatpipe 26 an das Wärmekoppelelement 34 kann durch einen thermisch leitenden Klebstoff unterstützt werden, wobei diese zusätzliche stoffschlüssige Verbindung die trotz des Formschlusses noch offenen Freiheitsgrade fixiert.

Über das Wärmekoppelelement 34 wird die von der Wärmequelle 24 (Elektronik 20, Beleuchtung 22) erzeugte Verlustwärme auf die Heatpipe 26, hier auf das distale Heatpipeende 28, übertragen. Bei dem erfindungsgemäßen Wärmeabfuhrmanagement ist nun vorgesehen, dass diese aufgenommene Verlustwärme von der Heatpipe 26 nicht zwingend stets bis zum proximalen Heatpipeende 30 geleitet und dort abgegeben wird, sondern das erfindungsgemäße Wärmeabfuhrmanagement beruht auf einer "flexiblen Wärmesenke", wie nachstehend erläutert wird.

Das Prinzip der "flexiblen Wärmesenke" beruht darauf, dass die Heatpipe 26 zwischen dem distalen Heatpipeende 28 und dem proximalen Heatpipeende 30 über zumindest eine Teillänge der Heatpipe 26 und über zumindest einen Teilumfang der Heatpipe 26 (unter Umfang ist der Umfang bezüglich der Längsrichtung der Heatpipe 26 zu verstehen) mit dem Schaft 12 flächig thermisch leitend gekoppelt ist, so dass Wärme von der Heatpipe 26 über zumindest eine Teillänge und zumindest einen Teilumfang des Schaftes 12 als Wärmesenke an die Umgebung abgeführt wird. In dem gezeigten Ausführungsbeispiel in Fig. 1 ist die Heatpipe 26 nahezu oder vollständig über die Gesamtlänge der Heatpipe 26 mit dem Schaft 12 thermisch leitend gekoppelt. Die Heatpipe 26 erstreckt sich dabei über mehr als die halbe Länge des Schaftes 12 und ist dabei über mehr als die halbe Länge des Schaftes 12 mit diesem thermisch leitend gekoppelt.

In dem Ausführungsbeispiel in Fig. 1 ist die Heatpipe 26 mit dem Schaft 12 vollumfänglich über einen Wärmesenkekörper 42 mit dem Schaft 12 thermisch leitend gekoppelt. Eine teilumfängliche Anbindung ist jedoch ebenso möglich. Der Wärmesenkekörper 42 steht dabei mit der Heatpipe 26 einerseits formschlüssig in Berührung und andererseits steht der Wärmesenkekörper 42 mit einer Innenseite 44 bzw. Innenwand des Schaftes 12 formschlüssig in Berührung.

Während in dem Ausführungsbeispiel in Fig. 1 die Heatpipe 26 über den Wärmesenkekörper 42 mit dem Schaft 12 thermisch leitend gekoppelt ist, ist es in anderen Ausführungsbeispielen, die später noch beschrieben werden, möglich, dass die Heatpipe 26 zumindest teilumfänglich unmittelbar mit der Innenseite 44 formschlüssig in Berührung steht und zusätzlich teilumfänglich über einen Wärmesenkekörper mit dem Schaft 12 thermisch leitend gekoppelt ist.

Das erfindungsgemäße Prinzip der "flexiblen Wärmesenke" bedeutet, dass sich die aktuelle Wärmesenke immer dort befindet, wo die geringste aktuelle Temperatur an der Oberfläche der Heatpipe 26 herrscht. Die Funktionsweise der Heatpipe 26 ist nicht darauf beschränkt, dass sich die Wärmesenke an einem der beiden Heatpipeenden, hier am proximalen Heatpipeende 30, befinden muss. Vielmehr sucht sich das Arbeitsmedium in der Heatpipe 26 den aktuell kältesten Punkt der Heatpipe 26 als Wärmesenke und den temporär wärmsten Punkt an der Heatpipe 26 als Wärmequelle. Der Ort der Wärmequelle 24 ist im vorliegenden Fall jedoch fest vorgegeben. Die Wärmesenke, also die kälteste Stelle entlang des Schaftes 12, ist nicht festgelegt. Durch die möglichst ganzheitliche Anbindung der Heatpipe 26 an den Schaft 12 in möglichst großflächiger, optimalerweise voller Länge kann sich die Wärmesenke überall entlang der Heatpipe 26 befinden. Entsprechend der Funktionsweise der Heatpipe 26 stellt sich also die Position der Wärmesenke flexibel entlang des Schaftes 12 ein. Hierdurch wird erreicht, dass sich die Wärme über die vorhandene Oberfläche des Schaftes 12 gleichmäßig verteilt und die Temperaturdifferenz somit so gering wie möglich gehalten wird.

In dem Ausführungsbeispiel in Fig. 1 ist der Wärmesenkekörper 42 insgesamt einstückig bzw. einteilig ausgebildet und erstreckt sich nahezu über die volle Länge der Heatpipe 26 zwischen einem distalen 46 und einem proximalen Ende 48 des Wärmesenkekörpers 42. Es versteht sich, dass der Wärmesenkekörper 42 einen oder mehrere in Längsrichtung durchgehende Öffnungen (nicht gezeigt) oder seitliche Längsaussparungen aufweist, die zur Durchführung von Stromversorgungs- und Datenübertragungsleitungen sowie ggfls. zur Durchführung von Lichtleitfasern geeignet sind.

Die formschlüssige Anbindung der Heatpipe 26 an den Wärmesenkekörper 42, die mit dem Bezugszeichen 50 versehen ist, sowie die formschlüssige Anbindung des Wärmesenkekörpers 42 an die Innenseite 44 des Schafts 12, wie mit 52 angedeutet, kann durch eine stoffschlüssige Fügung wie Kleben oder mit Lot fixiert sein, wobei das Fügematerial thermisch gut leitend sein muss. Ist jedoch eine unterschiedliche Längenausdehnung zwischen Heatpipe 26, Wärmesenkekörper 42 und/oder Schaft 12 zu berücksichtigen, entfällt eine solche zusätzliche stoffschlüssige Verbindung ganz oder teilweise.

Am proximalen Schaftende 16 sind in Fig. 1 des Weiteren ein Anschluss 54 für die Spannungsversorgung und Datenübertragung für die Elektronik 20 und ein Anschluss 56 für die Beleuchtung, beispielsweise zum Anschließen eines Lichtleitkabels, gezeigt.

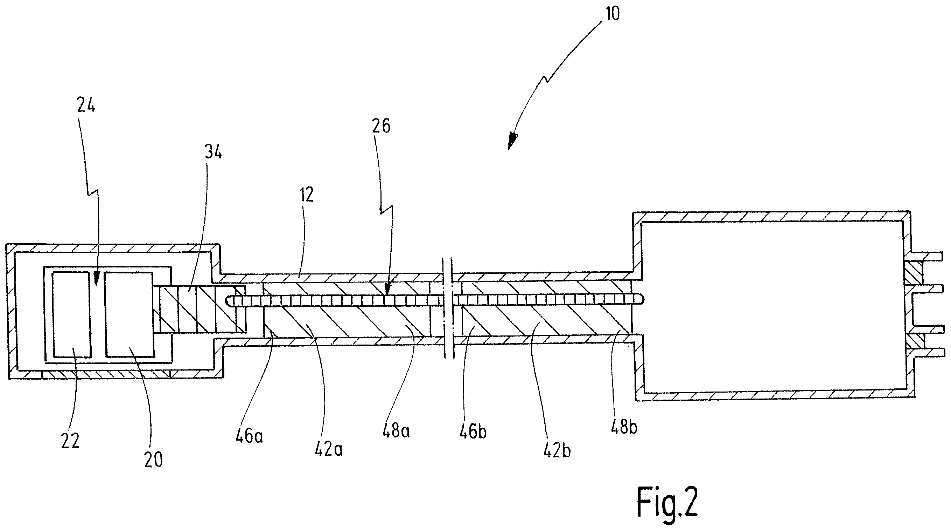

Mit Bezug auf Fig. 2 bis 13 werden nachfolgend weitere Ausführungsbeispiele beschrieben, wobei in Fig. 2 bis 13 Elemente, die mit Elementen in Fig. 1 identisch oder zu diesen ähnlich sind, mit den gleichen Bezugszeichen, ggf. ergänzt um Buchstaben, versehen sind.

Nachfolgend werden nur die jeweiligen Unterschiede zu dem Ausführungsbeispiel in Fig. 1 beschrieben.

Bei dem Instrument in Fig. 2 ist die Heatpipe 26 über mehrere, hier zwei Wärmsenkekörper 42a und 42b mit dem Schaft 12 thermisch leitend gekoppelt, wobei die Wärmesenkekörper 42a und 42b entlang der Heatpipe 26 verteilt und voneinander beabstandet angeordnet sind. Der Wärmesenkekörper 42a weist ein distales Ende 46a und ein proximales Ende 48a und der Wärmesenkekörper 42b ein distales Ende 46b und ein proximales Ende 48b auf.

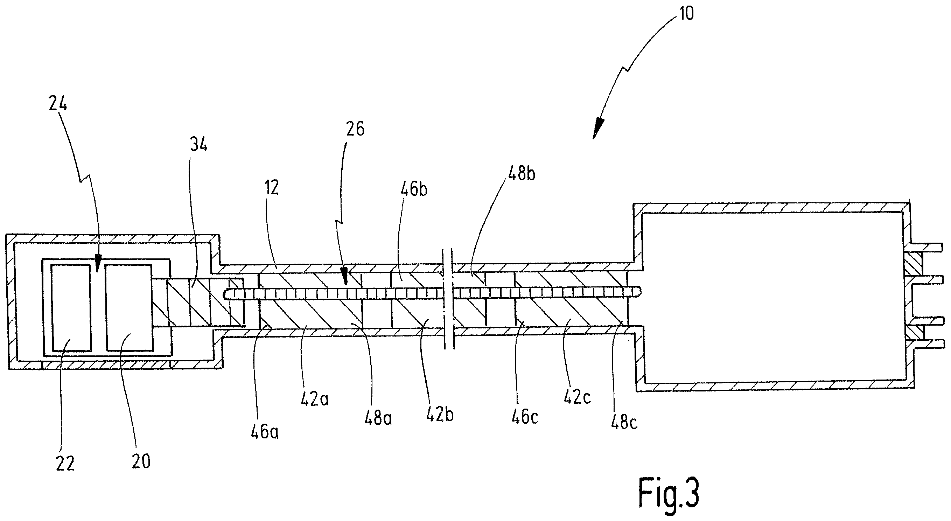

Das Instrument 10 gemäß Fig. 3 weist ebenfalls eine Mehrzahl an Wärmesenkekörpern, hier drei Wärmesenkekörper 42a, 42b und 42c auf, die entlang der Heatpipe 26 verteilt und voneinander beabstandet angeordnet sind und die die Heatpipe 26 mit dem Schaft 12 thermisch leitend koppeln. Der Wärmesenkekörper 42a weist ein distales Ende 46a und ein proximales Ende 48a, der Wärmesenkekörper 42b ein distales Ende 46b und ein proximales Ende 48b und der Wärmesenkekörper 42c ein distales Ende 46c und ein proximales Ende 48c auf.

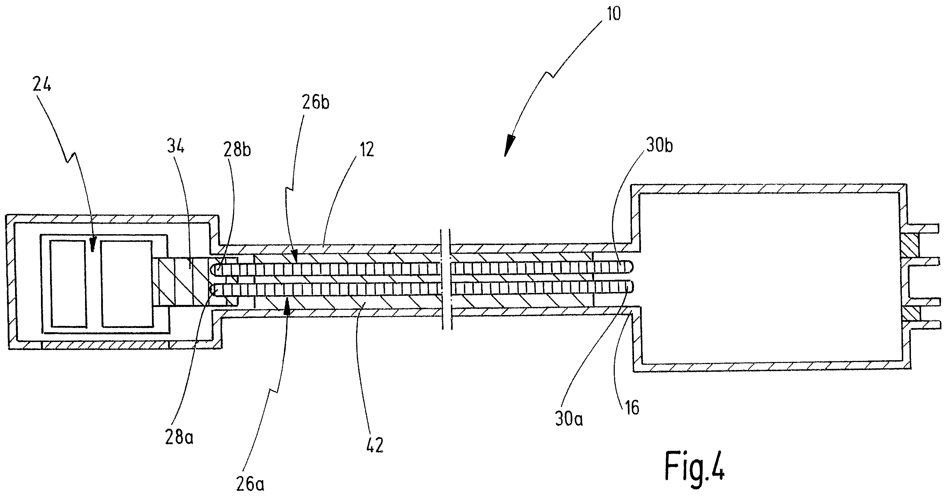

Das Instrument 10 in Fig. 4 weist im Unterschied zu den vorherigen Ausführungsbeispielen zwei Heatpipes 26a und 26b auf, die beide einerseits über das Wärmekoppelelement 34 an die Wärmequelle 24 thermisch leitend angebunden und andererseits an den Schaft 12 thermisch leitend angebunden sind, wobei dies über den Wärmesenkekörper 42, der hier wieder einteilig ausgebildet ist, erfolgt.

Die beiden Heatpipes 26a und 26b sind quer zur Längsrichtung des Schaftes 12 gesehen nebeneinander angeordnet und erstrecken sich beide vom Wärmekoppelelement 34 bis in den Bereich des proximalen Schaftendes 16. Die Heatpipe 26a weist ein distales Heatpipeende 28a und ein proximales Heatpipeende 30a und die Heatpipe 26b ein distales Heatpipeende 28b und ein proximales Heatpipeende 30b auf.

Wie bereits oben erwähnt, ist die Verwendung mehrerer dünner Heatpipes gegenüber der Verwendung nur einer Heatpipe mit größerem Querschnitt bevorzugt, zum einen wegen der verringerten Lageabhängigkeit der Heatpipes in Bezug auf Gravitation und des vergrößerten Oberflächen-Volumen-Verhältnisses.

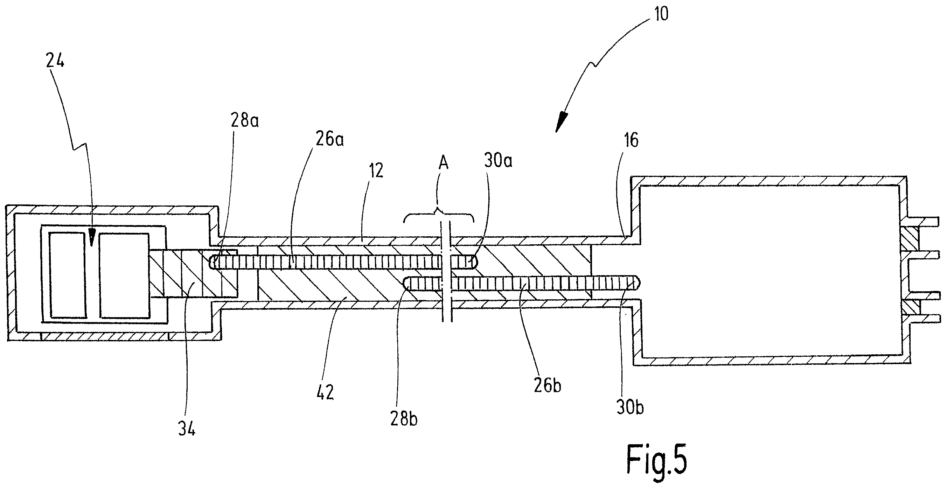

Das Instrument 10 in dem Ausführungsbeispiel in Fig. 5 weist ebenfalls zwei Heatpipes 26a und 26b auf, wobei in diesem Ausführungsbeispiel jedoch nur die Heatpipe 26a mit dem Wärmekoppelelement 34 thermisch leitend gekoppelt ist. Die Heatpipe 26a weist ein distales Heatpipeende 28a und ein proximales Heatpipeende 30a auf.

Die zweite Heatpipe 26b weist ein distales Heatpipeende 28b und ein proximales Heatpipeende 30b auf. Beide Heatpipes 26a und 26b erstrecken sich jeweils nur über einen gegenüber den vorherigen Ausführungsbeispielen kleineren Teillängenbereich des Schafts 12, zusammen erstrecken sich die beiden Heatpipes 26a und 26b jedoch über den gleichen Bereich wie in den vorherigen Ausführungsbeispielen. Die beiden Heatpipes 26a und 26b weisen einen Überlappungsbereich A mit thermisch leitender Kopplung aneinander auf, so dass zwischen die beiden Heatpipes 26a und 26b in diesem Bereich A ein Wärmeübergang stattfinden kann.

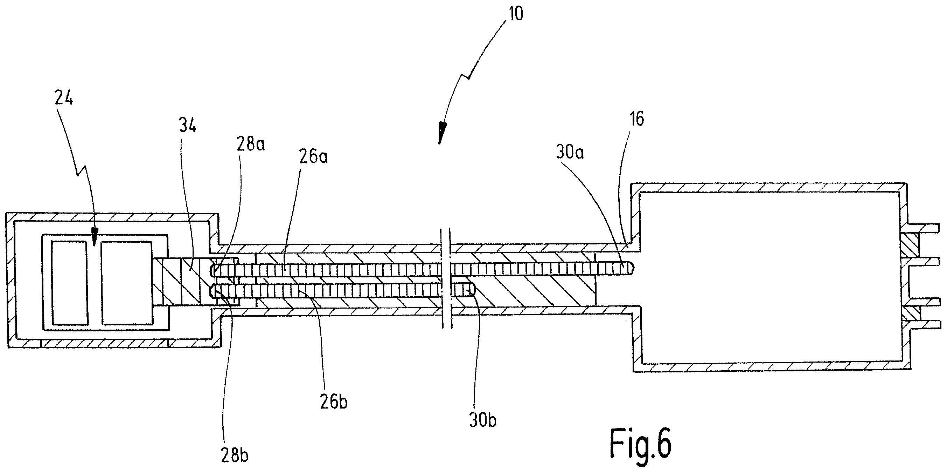

Das Instrument 10 in Fig. 6 weist ebenfalls zwei Heatpipes 26a und 26b auf, wobei beide Heatpipes 26a und 26b über das Wärmekoppelelement 34 mit der Wärmequelle 24 thermisch leitend gekoppelt sind. Während sich die Heatpipe 26a vom Wärmekoppelelement 34 bis zum proximalen Endbereich des Schaftes 12 erstreckt, ist die Heatpipe 26b kürzer ausgebildet und endet mit ihrem proximalen Heatpipeende 30b im Abstand zu dem proximalen Heatpipeende 30a.

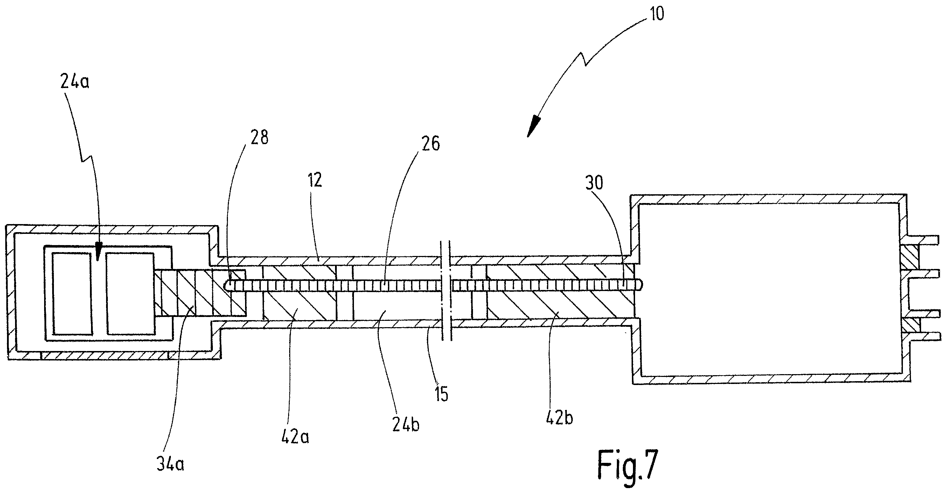

Während bei den bisherigen Ausführungsbeispielen nur eine Wärmequelle, und zwar die Wärmequelle 24 im distalen Endbereich des Schafts 12 vorhanden ist, weist das Instrument 10 in Fig. 7 zwei Wärmequellen auf, und zwar eine erste Wärmequelle 24a, die der Wärmequelle 24 in den bisherigen Ausführungsbeispielen entspricht, und eine zweite Wärmequelle 24b, die sich in einem mittleren Abschnitt 15 des Schafts 12 zwischen dem distalen Schaftende 14 und dem proximalen Schaftende 16 befindet. Die zusätzliche Wärmequelle 24b kann beispielsweise durch weitere elektronische Komponenten im mittleren Abschnitt 15 des Schafts 12 verursacht sein. Die Heatpipe 26 ist mit dieser zusätzlichen Wärmequelle 24b thermisch gekoppelt, um von der Wärmequelle 24b Wärme aufzunehmen und zu einer kälteren Stelle entlang des Schaftes 12 abzuführen. Auch hier gilt das Prinzip der "flexiblen Wärmesenke". Je nachdem wo sich die aktuell kälteste Stelle des Schaftes 12 befindet, wird die von der Wärmequelle 24b aufgenommene Wärme nach distal oder nach proximal abgeführt und an der aktuell kältesten Stelle an die Umgebung abgegeben.

Bei dem Ausführungsbeispiel in Fig. 7 ist die Heatpipe 26 über zwei Wärmesenkekörper 42a und 42b mit dem Schaft 12 thermisch leitend gekoppelt.

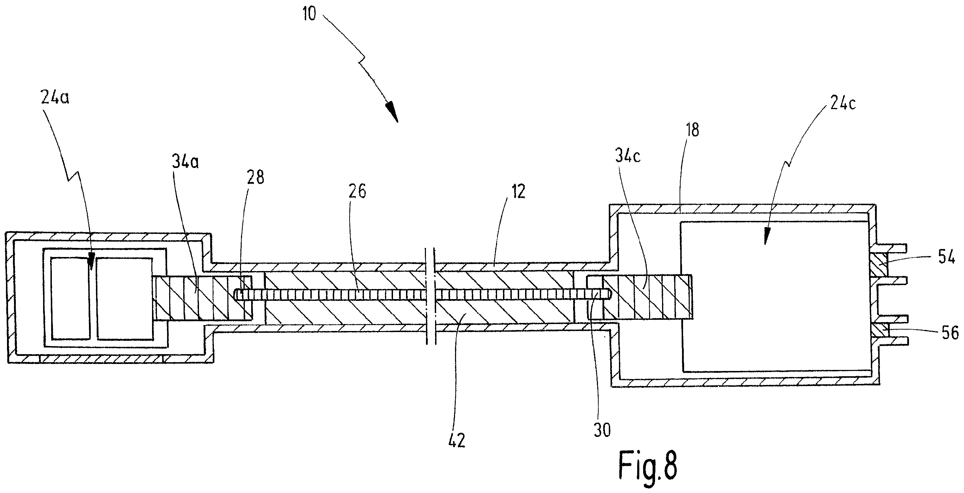

Das Instrument 10 in Fig. 8 weist ebenfalls zwei Wärmequellen 24a und 24c auf. Die Wärmequelle 24a befindet sich im distalen Endbereich des Schaftes 12, und die Wärmequelle 24c befindet sich im proximalen Endbereich des Schaftes 16 bzw im Instrumentenkopfgehäuse 18. Diese proximale Wärmequelle 24c kann durch im Instrumentenkopfgehäuse 18 vorhandene elektronische Bauelemente der Leistungselektronik, Datenverarbeitung, oder auch der Anschluss- und Koppelstellen 54 und 56 verursacht sein. Die Heatpipe 26 ist mit der zusätzlichen Wärmequelle 24c über ein Wärmekoppelelement 34c thermisch leitend gekoppelt, und mit der distalen Wärmequelle 24a über ein Wärmekoppelelement 34a. Die Wärmekoppelelemente 34a und 34c können wie das Wärmekoppelelement 34 gemäß der obigen Beschreibung ausgebildet sein, wobei eine Ausbildung des Wärmekoppelelements 34c als bewegliches Lager entfällt.

Auch bei dem Ausführungsbeispiel in Fig. 8 gilt das Prinzip der "flexiblen Wärmesenke", wobei die Heatpipe 26 die von der Wärmequelle 24a aufgenommene Wärme nach proximal zu einer aktuell kältesten Stelle des Schaftes 12 abführt, und die von der Wärmequelle 24c aufgenommene Wärme nach distal zu einer aktuell kältesten Stelle des Schaftes 12 abführt.

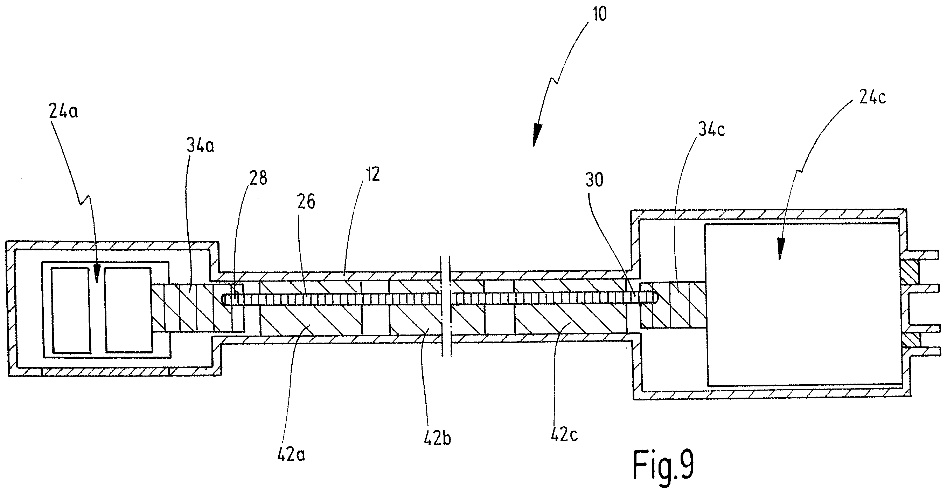

Das Ausführungsbeispiel gemäß Fig. 9 entspricht dem Ausführungsbeispiel in Fig. 8, wobei anstelle eines einteiligen Wärmesenkekörpers 42 in Fig. 8 eine Mehrzahl von Wärmesenkekörpern 42a, 42b, 42c wie bei dem Ausführungsbeispiel in Fig. 3 vorhanden sind.

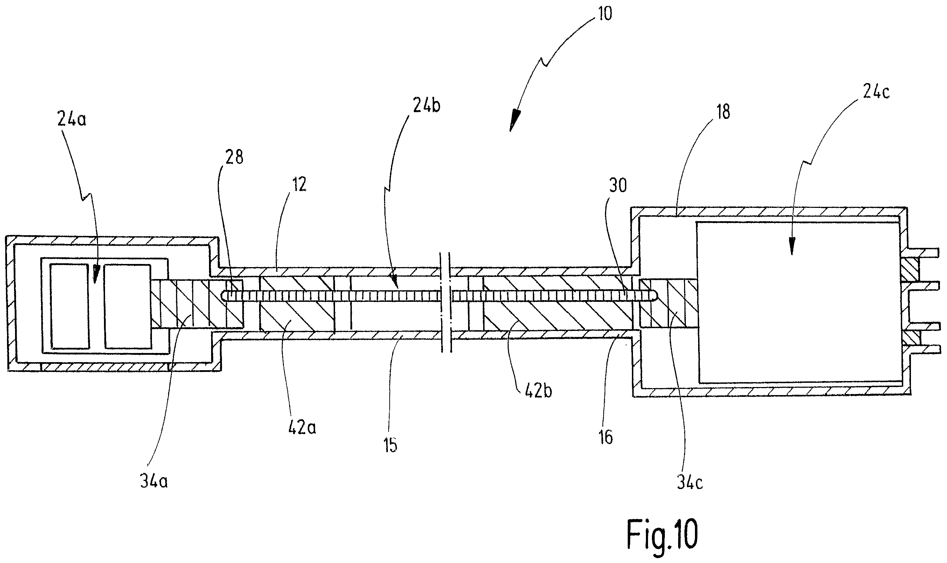

Fig. 10 zeigt ein Ausführungsbeispiel des Instruments 10, bei dem insgesamt drei Wärmequellen 24a, 24b und 24c vorhanden sind, entsprechend einer Kombination der Ausführungsbeispiele in Fig. 7 und 9. Die Wärmequelle 24a befindet sich im distalen Endbereich des Schafts 12, die Wärmequelle 24b in dem mittleren Abschnitt 15 des Schafts 12 und die Wärmequelle 24c im proximalen Endbereich des Schafts 12 bzw. im Instrumentenkopf 18. Das Prinzip der "flexiblen Wärmesenke" gilt auch für dieses Ausführungsbeispiel.

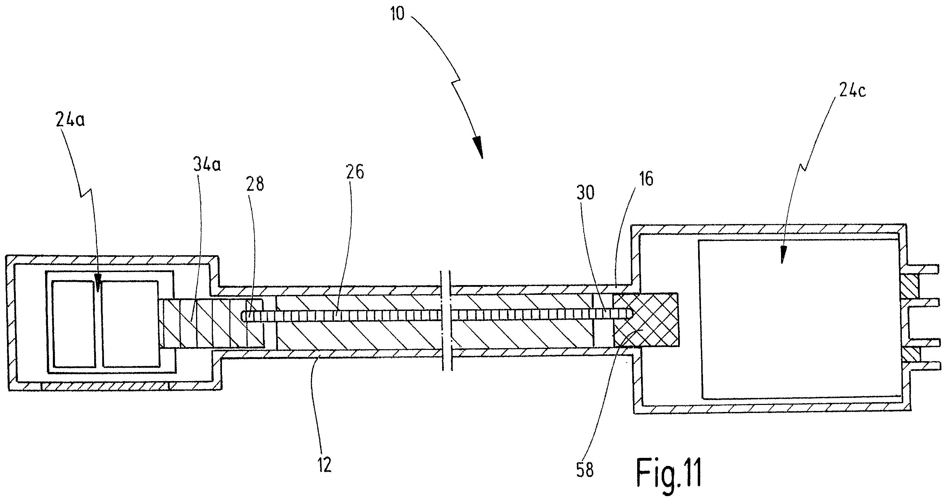

Das Ausführungsbeispiel in Fig. 11 unterscheidet sich von den bisherigen Ausführungsbeispielen dadurch, dass die Heatpipe 26 im proximalen Endbereich des Schaftes 12 gegenüber dem Schaft 12 mit einer thermischen Isolierung 58 thermisch isoliert ist. Diese Ausgestaltung ist für den Fall vorgesehen, dass beispielsweise die Verlustwärme der proximalen Wärmequelle 24c nur nach proximal abgegeben werden soll. Dies ist beispielsweise der Fall, wenn an verschiedenen Oberflächen entlang des Instruments 10 unterschiedliche Temperaturen zulässig sind.

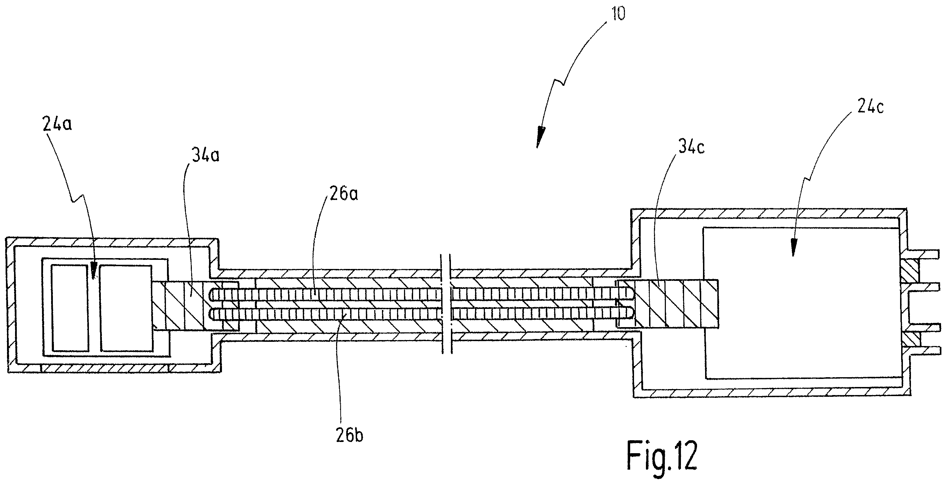

Fig. 12 zeigt ein Ausführungsbeispiel des Instruments 10, das eine Kombination der Ausführungsbeispiele in Fig. 4 und 8 darstellt, d.h. bei diesem Ausführungsbeispiel weist das Instrument 10 die beiden Wärmequellen 24a und 24c sowie die beiden Heatpipes 26a und 26b auf, die beide sowohl mit der distalen Wärmequelle 24a als auch mit der proximalen Wärmequelle 24c thermisch leitend gekoppelt sind.

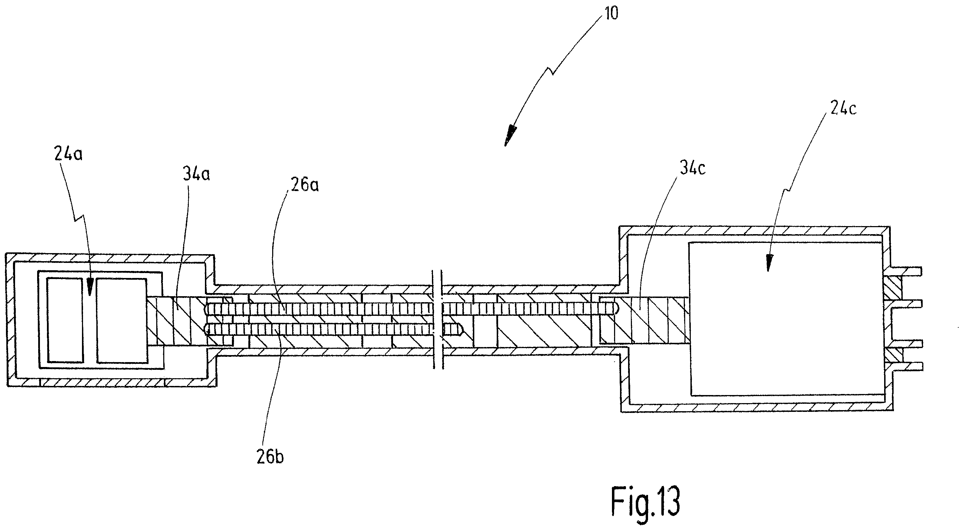

Fig. 13 zeigt das Instrument 10 in einem Ausführungsbeispiel, das eine Kombination der Ausführungsbeispiele in Fig. 6 und 8 darstellt. Bei diesem Ausführungsbeispiel sind die beiden Heatpipes 26a und 26b beide gemeinsam mit der distalen Wärmequelle 24a thermisch leitend gekoppelt, während mit der proximalen Wärmequelle 24c nur die Heatpipe 26a thermisch leitend gekoppelt ist. Eine solche Ausgestaltung kann in Betracht gezogen werden, wenn die distale Wärmequelle 24a eine größere Verlustwärmemenge erzeugt als die proximale Wärmequelle 24c. Durch die Anbindung beider Heatpipes 26a und 26b an die Wärmequelle 24a kann von dieser die höhere Verlustwärmemenge wirksamer abgeführt werden, während für die Abfuhr der Verlustwärme von der Wärmequelle 24b die eine Heatpipe 26a ausreichend ist.

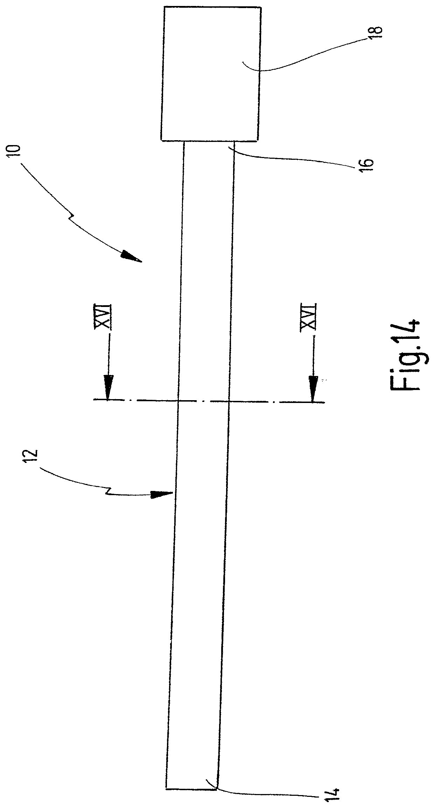

Mit Bezug auf Fig. 14 bis 17 werden weitere Ausführungsbeispiele beschrieben.

Fig. 14 zeigt zunächst schematisch das optische medizinische Instrument 10, mit dem langerstreckten Schaft 12 zwischen dem distalen Ende 14 und dem proximalen Ende 16 sowie dem Instrumentenkopfgehäuse 18.

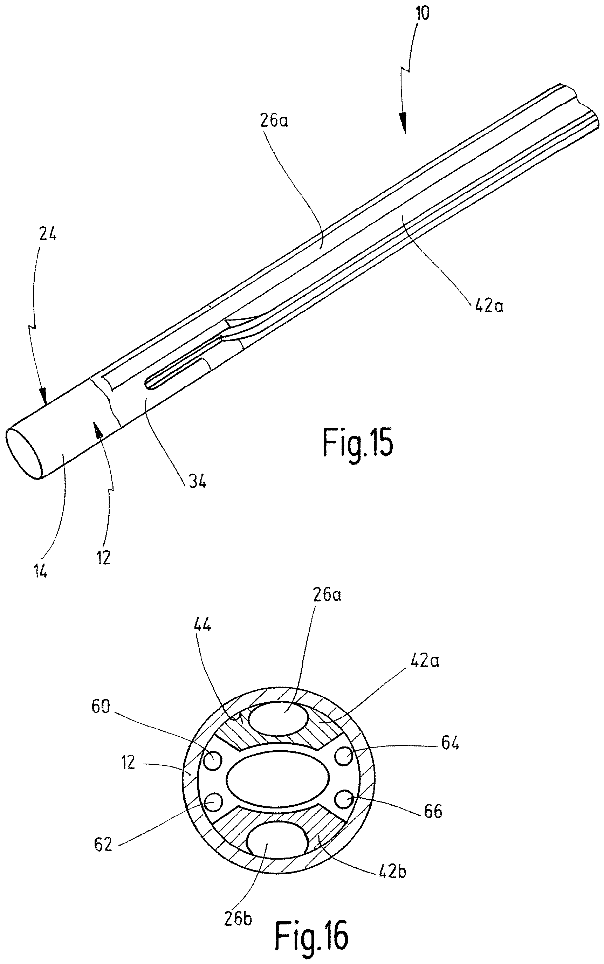

In Fig. 15 ist das Instrument 10 ausschnittsweise gezeigt, wobei außerdem der Schaft 12 über einen größeren Teilbereich seiner Längserstreckung aufgebrochen ist. Die Wärmequelle 24 befindet sich im distalen Endbereich des Schaftes 12. In Fig. 15 ist die erste Heatpipe 26a, das Wärmekoppelelement 34 und der Wärmesenkekörper 42a zu sehen.

Fig. 16 zeigt einen Querschnitt durch die Anordnung in Fig. 15 in einem Schnitt entlang der Linie XVI-XVI in Fig. 14. In dem Schnitt gemäß Fig. 16 ist auch die zweite Heatpipe 26b und der zweite Wärmesenkekörper 42b zu sehen. Bei dem Ausführungsbeispiel gemäß Fig. 15 und 16 sind die Heatpipes 26a und 26b jeweils über einen Teilumfangsbereich unmittelbar mit der Innenseite 44 des Schaftes 12 thermisch leitend unter Formschluss in Berührung, und weiterhin sind die beiden Heatpipes 26a und 26b formschlüssig mit dem jeweiligen Wärmesenkekörper 42a und 42b verbunden. Die Heatpipes 26a und 26b sind dabei in die Wärmesenkekörper 42a und 42b eingepresst, wodurch der Formschluss realisiert ist. Durch das Einpressen der Heatpipe 26a und 26b sind diese im Querschnitt geringfügig verformt, wie in Fig. 16 gezeigt ist. Wie in Fig. 16 ebenfalls gezeigt ist, erstrecken sich die Wärmesenkekörper 42a und 42b nur teilumfänglich entlang der Innenseite 44 des Schafts 12, so dass zwischen den Wärmesenkekörpern 42a und 42b Bereiche im Schaft 12 frei bleiben, durch die stromführende Leitungen, lichtführende Leitungen oder dgl. durchgeführt werden können, wie mit 60, 62, 64, 66 anggedeutet ist.

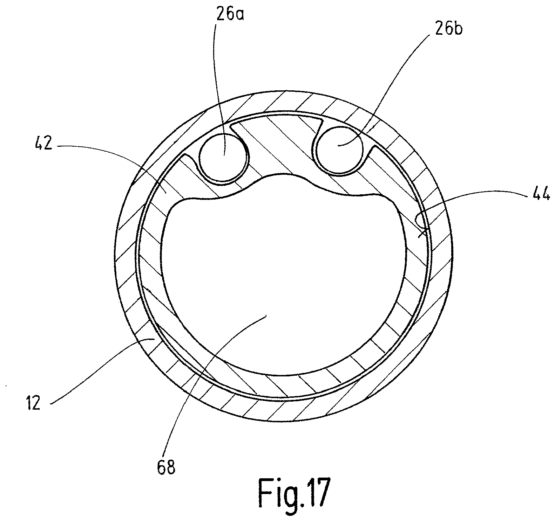

Fig. 17 zeigt eine zu Fig. 16 alternative Ausführungsform der Ausgestaltung und Anordnung der beiden Heatpipes 26a und 26b sowie des in diesem Ausführungsbeispiel vollumfänglich ausgebildeten Wärmesenkekörpers 42. Auch bei dem Ausführungsbeispiel in Fig. 17 stehen die Heatpipes 26a und 26b teilumfänglich unmittelbar mit der Innenseite 44 des Schaftes 12 in Berührung, und im Übrigen stehen die Heatpipes 26a und 26b formschlüssig mit dem Wärmesenkekörper 42 thermisch leitend in Berührung. Der Wärmesenkekörper 42 koppelt die Heatpipes 26a und 26b vollumfänglich an die Innenseite 44 des Schafts 12.

Der Wärmesenkekörper 42 umschließt einen Innenbereich 68, der zur Durchführung von Leitungen genutzt werden kann. Die Heatpipes 26a und 26b sind in diesem Ausführungsbeispiel nicht in den Wärmesenkekörper eingepreßt, sondern lediglich eingesteckt und daher im Querschnitt nicht verformt.

Es versteht sich, dass die mit Bezug auf die Ausführungsbeispiele in Fig. 1 bis 13 beschriebenen Aspekte auch bei den Ausführungsbeispielen in Fig. 14 bis 17 verwendet werden können, sowie umgekehrt.

## Patentansprüche

1. Optisches medizinisches Instrument, insbesondere Endoskop oder Exoskop, mit einem langerstreckten rohrförmigen thermisch leitenden Schaft (12), mit einer Verlustwärme erzeugenden Wärmequelle (24; 24a; 24 b; 24c), mit einer Heatpipe (26; 26a; 26b), die sich im Inneren des Schaftes (12) in Längsrichtung des Schaftes (12) erstreckt und ein distales Heatpipeende (28; 28a; 28b) und ein proximales Heatpipeende (30; 30a; 30b) aufweist, wobei die Heatpipe (26; 26a; 26b) mit der Wärmequelle (24; 24a; 24 b; 24c) thermisch gekoppelt ist, um von dieser Wärme aufzunehmen und von der Wärmequelle (24; 24a; 24b; 24c) abzuführen, wobei die Heatpipe (26; 26a; 26b) über zumindest eine Teillänge der Heatpipe (26; 26a; 26b) und über zumindest einen Teilumfang der Heatpipe (26; 26a; 26b) mit dem Schaft (12) flächig thermisch leitend gekoppelt ist, um Wärme von der Heatpipe (26; 26a; 26b) über zumindest eine Teillänge und über zumindest einen Teilumfang des Schaftes (12) an die Umgebung abzuführen, **dadurch gekennzeichnet, dass** sich die Heatpipe (26; 26a; 26b) zwischen dem distalen Heatpipeende (28, 28a, 28b) und dem proximalen Heatpipeende (30,30a,30b) zumindest über die halbe Länge des Schaftes (12) erstreckt und über zumindest die halbe Länge des Schaftes (12) mit diesem thermisch leitend gekoppelt ist.

2. Instrument nach Anspruch 1, **dadurch gekennzeichnet, dass** die Heatpipe (26; 26a; 26b) über zumindest die halbe Gesamtlänge der Heatpipe (26; 26a; 26b) mit dem Schaft (12) thermisch leitend gekoppelt ist.

3. Instrument nach Anspruch 1 oder 2, **dadurch gekennzeichnet, dass** die Heatpipe (26; 26a; 26b) nahezu oder vollständig über die Gesamtlänge der Heatpipe (26; 26a; 26b) mit dem Schaft (12) thermisch leitend gekoppelt ist.

4. Instrument nach einem der Ansprüche 1 bis 3, **dadurch gekennzeichnet, dass** die Heatpipe (26a; 26b) zumindest zum Teil unmittelbar mit dem Schaft (12) thermisch gekoppelt ist, wobei die Heatpipe (26a; 26b) zumindest teilumfänglich, vorzugsweise formschlüssig, mit einer Innenseite (44) des Schaftes (12) in Berührung steht.

5. Instrument nach einem der Ansprüche 1 bis 4, **dadurch gekennzeichnet, dass** die Heatpipe (26; 26a; 26b) zumindest teilumfänglich über einen Wärmesenkekörper (42; 42a; 42b; 42c) mit dem Schaft (12) thermisch leitend gekoppelt ist, wobei der Wärmesenkekörper (42; 42a; 42b; 42c) mit der Heatpipe (26; 26a; 26b) und mit einer Innenseite (44) des Schaftes (12) jeweils zumindest teilumfänglich formschlüssig in Berührung steht.

6. Instrument nach einem der Ansprüche 1 bis 5, **dadurch gekennzeichnet, dass** die Heatpipe (26; 26a; 26b) abschnittsweise über eine Mehrzahl an Wärmesenkekörpem (42a; 42b; 42c) mit dem Schaft (12) thermisch leitend gekoppelt ist, wobei die Wärmesenkekörper (42a; 42b; 42c) entlang der Heatpipe (26; 26a; 26b) verteilt angeordnet sind.

7. Instrument nach einem der Ansprüche 1 bis 6, **dadurch gekennzeichnet, dass** die Heatpipe (26; 26a; 26b) mit der Wärmequelle (24; 24a; 24c)über ein thermisch leitendes Wärmekoppelelement (34; 34a; 34c) verbunden ist, mit dem die Heatpipe (26; 26a; 26b) vorzugsweise formschlüssig verbunden ist.

8. Instrument nach Anspruch 7, **dadurch gekennzeichnet, dass** die Heatpipe (26; 26a; 26b) in das Wärmekoppelelement (34; 34a; 34c) eingepresst ist.

9. Instrument nach Anspruch 7 oder 8, **dadurch gekennzeichnet, dass** die Heatpipe (26; 26a; 26b) mittels eines thermisch leitenden Fügemittels in oder an dem Wärmekoppelelement (34; 34a; 34c) zusätzlich fixiert ist.

10. Instrument nach einem der Ansprüche 7 bis 9, **dadurch gekennzeichnet, dass** das Wärmekoppelement (34; 34a) als thermisch leitendes Lager mit zumindest einem Bewegungsfreiheitsgrad zur Wärmequelle (24; 24a) ausgebildet ist.

11. Instrument nach einem der Ansprüche 1 bis 10, **dadurch gekennzeichnet, dass** die Wärmequelle (24; 24a; 24 b; 24c) in einem distalen Endbereich des Schaftes (12), in einem mittleren Abschnitt (15) des Schaftes (12) oder in einem proximalen Endbereich des Schaftes (12) angeordnet ist.

12. Instrument nach Anspruch 11, **dadurch gekennzeichnet, dass** die Heatpipe (26; 26a; 26b) mit dem distalen Heatpipeende (28; 28a; 28b), mit einem mittleren Abschnitt zwischen dem distalen Heatpipeende (28; 28a; 28b) und dem proximalen Heatpipeende (30; 30a; 30b), oder mit dem proximalen Heatpipeende (30; 30a; 30b) mit der Wärmequelle (24; 24a; 24 b; 24c) thermisch gekoppelt ist.

13. Instrument nach einem der Ansprüche 1 bis 10, **dadurch gekennzeichnet, dass** die Wärmequelle (24; 24a; 24 b; 24c) eine erste Wärmequelle (24; 24a) ist, und dass zumindest eine zweite Wärmequelle (24b; 24c) vorhanden ist, die von der ersten Wärmequelle (24; 24a) in Längsrichtung des Schaftes (12) beabstandet ist, wobei die Heatpipe (26; 26a; 26b) mit der ersten und der zumindest einen zweiten Wärmequelle (24; 24a; 24 b; 24c) thermisch gekoppelt ist.

14. Instrument nach einem der Ansprüche 1 bis 13, **dadurch gekennzeichnet, dass** die Heatpipe (26; 26b) eine erste Heatpipe (26a) und zumindest eine zweite Heatpipe (26b) aufweist, wobei zumindest eine der zumindest zwei Heatpipes (26a; 26b) mit der Wärmequelle (24; 24a; 24 b; 24c) thermisch gekoppelt ist.

15. Instrument nach einem der Ansprüche 1 bis 14, **dadurch gekennzeichnet, dass** die Heatpipe (26; 26a; 26b) eine gesinterte Heatpipe ist.

## Claims

1. Optical medical instrument, in particular endoscope or exoscope, comprising an elongate tubular shaft (12), a heat source (24; 24a; 24b; 24c) generating lost heat, a heat pipe (26; 26a; 26b) extending inside the shaft (12) in the longitudinal direction of the shaft (12) and having a distal heat pipe end (28; 28a; 28b) and a proximal heat pipe end (30; 30a; 30b), wherein the heat pipe (26; 26a; 26b) is thermally coupled to the heat source (24; 24a; 24b; 24c) in order to collect heat from the latter and remove the heat from the heat source (24; 24a; 24b; 24c), wherein the heat pipe (26; 26a; 26b) is coupled thermally conductively and two-dimensionally to the shaft (12) over at least a partial length of the heat pipe (26; 26a; 26b) and over at least a partial circumference of the heat pipe (26; 26a; 26b), in order to remove heat from the heat pipe (26; 26a; 26b) over at least a partial length and over at least a partial circumference of the shaft (12) to the environment, **characterized in that** the heat pipe (26; 26a; 26b)extends, between the distal heat pipe end (28; 28a; 28b) and the proximal heat pipe end (30; 30a; 30b), over at least half the length of the shaft (12) and is coupled thermally conductively to the shaft (12) over at least half the length of the shaft (12).

2. Instrument of Claim 1, **characterized in that** the heat pipe (26; 26a; 26b) is coupled thermally conductively to the shaft (12) over at least half the total length of the heat pipe (26; 26a; 26b).

3. Instrument of Claim 1 or 2, **characterized in that** the heat pipe (26; 26a; 26b) is coupled thermally conductively to the shaft (12) almost or completely over the total length of the heat pipe (26; 26a; 26b).

4. Instrument of any one of Claims 1 through 3, **characterized in that** the heat pipe (26a; 26b) is thermally coupled at least in part directly to the shaft (12), wherein the heat pipe (26a; 26b) is in contact with an inner face (44) of the shaft (12) at least over part of the circumference, preferably with a form fit.

5. Instrument of any one of Claims 1 through 4, **characterized in that** the heat pipe (26; 26a; 26b) is coupled thermally conductively to the shaft (12) over at least part of the circumference via a heat sink body (42; 42a; 42b; 42c), wherein the heat sink body (42; 42a; 42b; 42c) is in form-fit contact with the heat pipe (26; 26a; 26b) and with an inner face (44) of the shaft (12), in each case over at least part of the circumference.

6. Instrument of any one of Claims 1 through 5, **characterized in that** the heat pipe (26; 26a; 26b) is coupled thermally conductively to the shaft (12) section-wise via a plurality of heat sink bodies (42a; 42b; 42c), wherein the heat sink bodies (42a; 42b; 42c) are distributed along the heat pipe (26; 26a; 26b).

7. Instrument of any one of Claims 1 through 6, **characterized in that** the heat pipe (26; 26a; 26b) is connected to the heat source (24; 24a; 24c) via a thermally conductive heat coupling element (34; 34a; 34c), to which the heat pipe (26; 26a; 26b) is preferably connected with a form fit.

8. Instrument of Claim 7, **characterized in that** the heat pipe (26; 26a; 26b) is pressed into the heat coupling element (34; 34a; 34c).

9. Instrument of Claim 7 or 8, **characterized in that** the heat pipe (26; 26a; 26b) is additionally fixed in or on the heat coupling element (34; 34a; 34c) by means of a thermally conductive joining means.

10. Instrument of any one of Claims 7 through 9, **characterized in that** the heat coupling element (34; 34a) is designed as a thermally conductive bearing with at least one degree of freedom of movement with respect to the heat source (24; 24a).

11. Instrument of any one of Claims 1 through 10, **characterized in that** the heat source (24; 24a; 24b; 24c) is arranged in a distal end area of the shaft (12), in a middle portion (15) of the shaft (12) or in a proximal end area of the shaft (12).

12. Instrument of Claim 11, **characterized in that** the heat pipe (26; 26a; 26b) is thermally coupled to the heat source (24; 24a; 24b; 24c) via the distal heat pipe end (28; 28a; 28b), via a middle portion between the distal heat pipe end (28; 28a; 28b) and the proximal heat pipe end (30; 30a; 30b), or via the proximal heat pipe end (30; 30a; 30b).

13. Instrument of any one of Claims 1 through 10, **characterized in that** the heat source (24; 24a; 24b; 24c) is a first heat source (24; 24a), and **in that** at least one second heat source (24b; 24c) is present which is at a distance from the first heat source (24; 24a) in the longitudinal direction of the shaft (12), wherein the heat pipe (26; 26a; 26b) is thermally coupled to the first and the at least one second heat source (24; 24a; 24b; 24c).

14. Instrument of any one of Claims 1 through 13, **characterized in that** the heat pipe (26; 26b) has a first heat pipe (26a) and at least one second heat pipe (26b), wherein at least one of the at least two heat pipes (26a; 26b) is thermally coupled to the heat source (24; 24a; 24b; 24c).

15. Instrument of any one of Claims 1 through 14, **characterized in that** the heat pipe (26; 26a; 26b) is a sintered heat pipe.

## Revendications

1. Instrument médical optique, en particulier endoscope ou exoscope, comprenant une tige (12) thermiquement conductrice de forme tubulaire étirée en longueur, comprenant une source de chaleur (24 ; 24a ; 24b ; 24c) produisant une perte de chaleur, comprenant un caloduc (26 ; 26a ; 26b), qui s'étend à l'intérieur de la tige (12) dans le sens longitudinal de la tige (12) et qui présente une extrémité de caloduc distale (28 ; 28a ; 28b) et une extrémité de caloduc proximale (30 ; 30a ; 30b), dans lequel le caloduc (26 ; 26a ; 26b) est couplé thermiquement à la source de chaleur (24 ; 24a ; 24b ; 24c) afin d'absorber la chaleur provenant de cette dernière et de l'évacuer de la source de chaleur (24 ; 24a ; 24b ; 24c), dans lequel le caloduc (26 ; 26a ; 26b) est couplé de manière conductrice thermiquement à plat à la tige (12) par l'intermédiaire d'au moins une longueur partielle du caloduc (26 ; 26a ; 26b) et par l'intermédiaire d'au moins une périphérie partielle du caloduc (26 ; 26a ; 26b) afin d'évacuer dans l'environnement de la chaleur du caloduc (26 ; 26a ; 26b) sur au moins une longueur partielle et sur au moins une périphérie partielle de la tige (12), **caractérisé en ce que** le caloduc (26; 26a; 26b) s'étend entre l'extrémité de caloduc distale (28, 28a, 28b) et l'extrémité de caloduc proximale (30, 30a, 30b) au moins sur la moitié de la longueur de la tige (12) et est couplé de manière conductrice thermiquement à la tige (12) sur au moins la moitié de la longueur de cette dernière.

2. Instrument selon la revendication 1, **caractérisé en ce que** le caloduc (26 ; 26a ; 26b) est couplé de manière conductrice thermiquement à la tige (12) sur au moins la moitié de la longueur totale du caloduc (26 ; 26a ; 26b).

3. Instrument selon la revendication 1 ou 2, **caractérisé en ce que** le caloduc (26 ; 26a ; 26b) est couplé de manière conductrice thermiquement à la tige (12) quasiment ou totalement sur la longueur totale du caloduc (26 ; 26a ; 26b).

4. Instrument selon l'une quelconque des revendications 1 à 3, **caractérisé en ce que** le caloduc (26a ; 26b) est couplé thermiquement au moins en partie directement à la tige (12), dans lequel le caloduc (26a ; 26b) est en contact au moins sur une partie de la périphérie, de préférence par complémentarité de forme, avec un côté intérieur (44) de la tige (12).

5. Instrument selon l'une quelconque des revendications 1 à 4, **caractérisé en ce que** le caloduc (26 ; 26a ; 26b) est couplé de manière conductrice thermiquement à la tige (12) au moins sur une partie de la périphérie par l'intermédiaire d'un corps dissipateur thermique (42 ; 42a ; 42b ; 42c), dans lequel le corps dissipateur thermique (42 ; 42a ; 42b ; 42c) est en contact respectivement au moins sur une partie de la périphérie par complémentarité de forme avec le caloduc (26 ; 26a ; 26b) et avec un côté intérieur (44) de la tige (12).

6. Instrument selon l'une quelconque des revendications 1 à 5, **caractérisé en ce que** le caloduc (26 ; 26a ; 26b) est couplé de manière conductrice thermiquement à la tige (12) par endroits par l'intermédiaire d'une multitude de corps dissipateurs de chaleur (42a ; 42b ; 42c), dans lequel les corps dissipateurs de chaleur (42a ; 42b ; 42c) sont disposés de manière répartie le long du caloduc (26 ; 26a ; 26b).

7. Instrument selon l'une quelconque des revendications 1 à 6, **caractérisé en ce que** le caloduc (26 ; 26a ; 26b) est relié à la source de chaleur (24 ; 24a ; 24c) par l'intermédiaire d'un élément de couplage thermique (34 ; 34 ; 34c) conducteur thermiquement, auquel le caloduc (26 ; 26a ; 26b) est relié de préférence par complémentarité de forme.

8. Instrument selon la revendication 7, **caractérisé en ce que** le caloduc (26 ; 26a ; 26b) est introduit par pression dans l'élément de couplage thermique (34 ; 34a ; 34c).

9. Instrument selon la revendication 7 ou 8, **caractérisé en ce que** le caloduc (26 ; 26a ; 26b) est fixé en supplément dans l'élément de couplage thermique (34 ; 34a ; 34c) ou au niveau de ce dernier au moyen d'un moyen d'assemblage conducteur thermiquement.

10. Instrument selon l'une quelconque des revendications 7 à 9, **caractérisé en ce que** l'élément de couplage thermique (34 ; 34a) est réalisé sous la forme d'un palier conducteur thermiquement présentant au moins un degré de liberté de mouvement par rapport à la source de chaleur (24 ; 24a).

11. Instrument selon l'une quelconque des revendications 1 à 10, **caractérisé en ce que** la source de chaleur (24 ; 24a ; 24b ; 24c) est disposée dans une zone d'extrémité distale de la tige (12), dans une section (15) centrale de la tige (12) ou dans une zone d'extrémité proximale de la tige (12).

12. Instrument selon la revendication 11, **caractérisé en ce que** le caloduc (26 ; 26a ; 26b) est couplé thermiquement à la source de chaleur (24 ; 24a ; 24b ; 24c) par l'extrémité de caloduc distale (28 ; 28a ; 28b), par une section centrale entre l'extrémité de caloduc distale (28 ; 28a ; 28b) et l'extrémité de caloduc proximale (30 ; 30a ; 30b) ou par l'extrémité de caloduc proximale (30 ; 30a ; 30b).

13. Instrument selon l'une quelconque des revendications 1 à 10, **caractérisé en ce que** la source de chaleur (24 ; 24a ; 24b ; 24c) est une première source de chaleur (24 ; 24a), et qu'au moins une deuxième source de chaleur (24b ; 24c) est présente, laquelle est espacée de la première source de chaleur (24; 24a) dans le sens longitudinal de la tige (12), dans lequel le caloduc (26 ; 26a ; 26b) est couplé thermiquement à la première et à l'au moins une deuxième source de chaleur (24 ; 24a ; 24b ; 24c).

14. Instrument selon l'une quelconque des revendications 1 à 13, **caractérisé en ce que** le caloduc (26 ; 26b) présente un premier caloduc (26a) et au moins un deuxième caloduc (26b), dans lequel au moins un des deux caloducs (26a ; 26b) ou plus est couplé thermiquement à la source de chaleur (24 ; 24a ; 24b ; 24c).

15. Instrument selon l'une quelconque des revendications 1 à 14, **caractérisé en ce que** le caloduc (26 ; 26a ; 26b) est un caloduc fritté.
